# EUROPEAN PATENT APPLICATION

(11) **EP 3 957 740 A1**
(43) Date of publication of application: **23.02.2022**
(21) Application number: 20790711.4
(22) Date of filing: 17.04.2020
(51) Int. Cl.: C12Q 1/02, C12Q 1/04, C12M 1/34

(54) **METHOD FOR MEASURING BACTERIAL INHIBITION OF ANTIBACTERIAL DRUG, AND BACTERIAL COUNTING DEVICE AND METHOD THEREOF**

(30) Priority: 18.04.2019 CN 201910315873; 12.12.2019 CN 201911290900
(71) Applicant: Beijing Xinji Jinnuo Medical Equipment Co., Ltd., Beijing 100176 (CN)
(72) Inventor: CUI, Jing, Beijing 100176 (CN); TANG, Mingzhong, Beijing 100176 (CN); ZHANG, Huicui, Beijing 100176 (CN); XU, Peng, Beijing 100176 (CN)
(74) Representative: HGF
(86) International application number: PCT/CN2020/085275
(87) International publication number: WO 2020/211835

(57) **Abstract**

Disclosed are a method for measuring the inhibition of bacteria by an antibacterial drug, and a bacterial counting device and method thereof. The inhibition measurement method includes: adding a predetermined concentration of antibacterial drug to bacteria to be detected and setting as a mixture of bacteria and drug, while also setting the bacteria to be detected without the antibacterial drug as a positive control; when a first predetermined duration is reached from the time when the antibacterial drug is added, obtaining the current number of the bacteria in the mixture of bacteria and drug mixture of bacteria and drug and the current number of the bacteria in the positive control; according to the ratio of the current number of the bacteria in the mixture of bacteria and drug mixture of bacteria and drug to the current number of the bacteria in the positive control, determining that the antibacterial drug at the predetermined concentration inhibits or partially inhibits or does not inhibit the bacteria. The method is suitable for rapid drug susceptibility testing.

## Description

### Technical Field

The present disclosure relates to the field of biopharmaceuticals, and specifically to a method for measuring inhibition of bacteria by an antibacterial drug, a bacterial counting device and method thereof.

### Background

The problem of drug resistance in bacteria is getting more and more serious while spreading rapidly and widely around the world, attracting the attention of governments of all countries, and a large number of regulatory regulations have been introduced in China as well. The proper use of antibiotics is the most essential task in dealing with drug resistance in bacteria, and rapid antibiotics susceptibility testing is a top priority.

With the development of mass spectrometry and nucleic acid technology, rapid identification of bacteria has been basically achieved (with results given on the same day and completed within 1-2 hours), therefore, the development of new rapid antibiotics susceptibility testing is more urgent and has practical significance. It is the cornerstone of antibiotics management to switch from empiric broad-spectrum antibiotic therapy to targeted therapy as soon as possible, but the current reporting time of drug susceptibility testing restricts clinical practice. Since the reporting time of conventional manual method is too long, fully automated drug susceptibility testing methods are mostly employed in current clinical practice, among which the fastest detecting systems are the VITEK system of BioMerieux (France) and the Phoenix system of BD (USA). Although the reliability and accuracy of these two systems have been proven, the average reporting time is 12.1 hours for Phoenix and 9.8 hours for Vitek2, which only allows doctors to select targeted medication the next day considering their daily workflow and schedules.

At present, in order to shorten the time for AST (antimicrobial susceptibility testing) reporting, a number of research has been conducted at home and abroad with a variety of methods being developed, such as mass spectrometry method, flow cytometry method, oscillating cantilever-based microbial cell weighing method, isothermal microcalorimetry method, magnetic bead rotation method, microfluidic droplet detection method, real-time PCR method, microarray method, conductivity method, surface plasmon resonance method, RNA sequencing method, bacteriophage method, real-time microscopy method and microcosmic sound wave method. However, these technologies are still at a research stage for small sample analysis only, which all require operation by professional technicians. Moreover, the equipments are expensive, non-conventional and specialized ones featuring complicated operation, unstable performance, high cost and inconvenience in use. Therefore, it is difficult to tell their practical prospects.

Rapid drug susceptibility testing is performed with two types of methods, i.e., phenotypic methods and non-phenotypic methods. Nonphenotypic methods are mainly nucleic acid-based molecular biology methods, such as real-time PCR method, microarray method, RNA sequencing method, transcriptome and whole genome sequencing, etc. Their advantages include: 1. short time, for example, multiple drug resistance genes can be detected in a multiplex PCR of positive blood cultures; 2. quantitative analysis can be achieved by digital PCR; 3. corresponding drug resistance mechanism can be clarified. The disadvantages include: 1. the complexity of the resistance mechanism of bacteria may lead to a heavy workload which affects the economy and rapidity if applied in clinical practice; 2. a great amount of verification is required for the consistency between test results of drug-resistance genes and the phenotype thereof because of genetic heterogeneity; 3. new drug-resistance mechanisms cannot be detected while acute detection of new drug-resistance mechanisms is urgently needed in clinical practice; 4. the methods, immature as they are, have not yet been applied in clinical practice and further clinical observation is required so as to be applied in clinical practice after standardization and being recognized by experts globally.

In a phenotypic drug susceptibility testing, the response of bacteria to a drug in vitro is observed directly, and the susceptibility and tolerance of bacteria to antibiotics can be observed directly. Conventional phenotypic drug susceptibility testing has been fully developed, validated, verified and well proven in clinical practice, and have become the reference standard for drug susceptibility testing methods The "intermediate techniques", i.e., those developed on the basis of conventional culture methods, can be implemented earlier with high feasibility, thus raising expectations before genetically based nonphenotypic drug susceptibility testing is improved and definitively established.

Additionally, cell counters are used in most of the drug susceptibility testing. A cell counter refers mostly to an instrument that measures the number of platelets, white blood cells, red blood cells, etc. Fully automated cell counters are widely used, the Coulter principle for analysis of particles, has always been the internationally recognized as standard control method for measuring cell and particle size and has always played an important role in hematological analysis.

The following problems exist in the prior art of bacterial counting devices and methods: ① There are no devices on the market yet that use resistance counting method for measuring the number of bacteria. (2) The diameter of the aperture of existing cell counters is suitable for measuring large cells like red blood cells and white blood cells to ensure that the cells can pass through the aperture one by one; bacteria are small and cannot pass through the aperture one by one normally, thus the number of the measured bacteria passing through the aperture at the same time may be greater than or equal to 2, resulting in inaccurate counting. ③ When measuring red blood cells or white blood cells, an aperture smaller than 50 µm may cause the clogging of the aperture of an existing counting instrument, therefore, the diameter of apertures in the prior art is limited to greater than 50 µm. ④ Manual operation: In the prior art, the number of bacteria can be determined easily and precisely by the use of microscopy, or through methods like staining, projection or photography to obtain pictures for measuring based on magnification, which is labor-intensive and time-consuming, so these counting methods are not widely promoted in clinical applications. ⑤ Manual operation: the size and shape of bacteria vary among species, for example, there are branched, filamentous, spindle-shaped and chained bacteria among others. It is difficult to determine the number of bacteria with the above-mentioned methods in the prior art if there are much overlapping of bacteria.

Therefore, it is particularly important to propose an economical and rapid scheme of susceptibility test, and to design an automated bacterial counting device specifically for bacteria which is fast, precise and convenient to use.

### Summary

The present disclosure is proposed to provide a method for measuring inhibition of bacteria by an antibacterial drug, a bacterial counting device and method thereof, to at least solve the technical problem of long turn-around time of measurement methods for bacterial inhibition of antibacterial drugs in the prior art, which only allows doctor to select targeted medication the next day.

According to one aspect of the present disclosure, a method is provided for measuring inhibition of bacteria by an antibacterial drug. The inhibition measurement method includes: adding a predetermined concentration of an antibacterial drug to bacteria to be detected and setting as a mixture of bacteria and drug; while also setting the bacteria to be detected without the antibacterial drug as a positive control; when a first predetermined duration is reached from the time when the antibacterial drug is added, obtaining the current number of the bacteria in the mixture of bacteria and drug and the current number of the bacteria in the positive control; determining that the antibacterial drug of the predetermined concentration inhibits or partially inhibits or does not inhibit the bacteria based on the ratio of the current number of the bacteria in the mixture of bacteria and drug to the current number of the bacteria in the positive control.

Optionally, when the ratio of the current number of the bacteria in the mixture of bacteria and drug to the current number of the bacteria in the positive control equals to the first predetermined threshold, it is determined that the antibacterial drug of the predetermined concentration inhibits the about antibacterial drug.

Optionally, the first predetermined threshold is any one value between 0 and 0.6.

Optionally, the first predetermined threshold is any one value between 0 and 0.4.

Optionally, when the ratio of the current number of the bacteria in the mixture of bacteria and drug to the current number of the bacteria in the positive control equals to a second predetermined threshold, it is determined that the antibacterial drug of the predetermined concentration partially inhibit the bacteria, but inhibition is not achieved;
when a second predetermined duration is reached from a time when the antibacterial drug is added, obtaining a second current number of the bacteria in the mixture of bacteria and drug and a second current number of the bacteria in the positive control, wherein the second predetermined duration is longer than the first predetermined duration;
when the ratio of the second current number of the bacteria in the mixture of bacteria and drug to the second current number of the bacteria in the positive control equals to the first predetermined threshold, it is determined that the antibacterial drug of the predetermined concentration inhibits the bacteria.

Optionally, when the ratio of the current number of the bacteria in the mixture of bacteria and drug to the current number of the bacteria in the positive control is greater than a second predetermined threshold, it is determined that the antibacterial drug of the predetermined concentration does not inhibit the bacteria.

Optionally, the first predetermined duration is any one value between 0 and 1.5 hours and the first predetermined duration does not equal to 0 hour.

Optionally, the second predetermined threshold is any one value between 0.4 and 0.8.

Optionally, a resistance counting method is used for obtaining the current number of the bacteria in the mixture of bacteria and drug and the current number of the bacteria in the positive control.

Optionally, the method for measuring inhibition of bacteria by an antibacterial drug includes the following measuring steps:
a. preparing the bacteria: inoculating the bacteria on a medium and incubating at a temperature between 20 degrees Celsius (°C) and 40 degrees Celsius (°C) for 15-24 hours and for later use;
b. preparing the mixture of bacteria and drug and the positive control, and incubating at a temperature between 20°C and 40°C.
c. after the first predetermined duration or the second predetermined duration, using the resistance counting method to obtain the current number or the second current number of the bacteria in the mixture of bacteria and drug, or the current number or the second current number of the bacteria in the positive control;
d. when the ratio of the current number or the second current number of the bacteria in the mixture of bacteria and drug to the current number or the second current number of the bacteria in the positive control equals to any one value between 0 and 0.4, it is determined that the antibacterial drug of the predetermined concentration inhibits the bacteria.

Optionally, in the step a, the bacteria is inoculated on a blood agar medium and incubated at 37 degrees Celsius (°C) for 18 hours; and/or
in the step b, the mixture of bacteria and drug and the positive control are prepared and incubated at 37°C; and/or
in the step c, the first predetermined duration is 0.5 hours or 1 hour or 1.5 hours; the second predetermined duration is 2 hours or 2.5 hours or 3 hours.

Optionally, the current number of the bacteria in the mixture of bacteria and drug and the current number of the bacteria in the positive control are obtained by using a flow cytometry method or a microscopic method for counting bacteria or a counter measuring method or an electric counter counting method or a live cell counting method or a cell weight measuring method.

According to another aspect of the present disclosure, a bacterial counting device is provided. The bacterial counting device may be used for obtaining the current number of the bacteria in the mixture of bacteria and drug and the current number of the bacteria in the positive control in the method for measuring inhibition of bacteria by an antibacterial drug. The bacterial counting device includes: a sampling module for obtaining a sample of bacteria to be counted; a counting cell module comprising: an aperture, a front cell, a rear cell and electrodes, wherein the front cell and the rear cell are connected through the aperture, one of the electrodes being on each side of the aperture, the liquid pressure between the front cell and the rear cell being vacuum, the vacuum being used to make the sample of bacteria to be counted to pass from the front cell through the aperture into the rear cell; and a circuit control system for enumeration of bacteria in the sample of bacteria to be counted based on a pulse signal when the pulse signal generated on both sides of the aperture is detected, wherein the pulse signal is used to indicate that a bacterium in the sample of bacteria to be counted has passed through the aperture.

Optionally, the circuit control system includes: a first processor for detecting the pulse signal, transmitting the pulse signal to a processing equipment, and obtaining the number of bacteria in the sample of bacteria to be counted sent by the processing equipment, wherein the number of bacteria in the sample of bacteria to be counted is obtained on the basis of bacterial feature data indicated by the pulse signal; or
a second processor for detecting the pulse signal and for enumeration of bacteria in the sample of bacteria to be counted on the basis of bacterial feature data indicated by the pulse signal.

Optionally, the circuit control system includes: a first power circuit for supplying a constant current to the aperture through the electrodes, wherein the pulse signal is a pulse signal triggered by one or more of the bacteria passing through the aperture when the constant current is supplied to the aperture; or
a second power circuit for supplying a constant voltage to the aperture through the electrodes, wherein the pulse signal is a pulse signal triggered by one or more of the bacteria passing through the aperture when the constant voltage is supplied to the aperture.

Optionally, the diameter of the aperture is a diameter within a first target diameter range, wherein the first target diameter range is used to allow only one bacterium to pass through the aperture at a time when bacteria in the sample of bacteria to be counted pass through the aperture;
the diameter of the aperture is a diameter within a second target diameter range, wherein the second target diameter range is used to allow a plurality of bacteria to pass through the aperture at a time when bacteria in the sample of bacteria to be counted pass through the aperture.

Optionally, when the diameter of the aperture is a diameter within the first target diameter range, the diameter of the aperture is 30 microns to 70 microns, and/or, the aperture has a length of 30 microns to 100 microns.

Optionally, when the diameter of the aperture is a diameter within the first target diameter range, the diameter of the aperture is 40 microns to 60 microns, and/or, the aperture has a length of 40 microns to 70 microns.

Optionally, when the diameter of the aperture is a diameter within the first target diameter range, the diameter of the aperture is 50 microns, and/or, the aperture has a length of 50 microns.

According to a further aspect of the present disclosure, a bacterial counting method is provided. The bacterial counting method may be used for obtaining the current number of the bacteria in the mixture of bacteria and drug and the current number of the bacteria in the positive control in the method for measuring inhibition of bacteria by an antibacterial drug. The bacterial counting method includes: adding a sample of bacteria to be counted into a counting cell module, wherein the counting cell module includes: an aperture, a front cell, a rear cell and electrodes, the front cell and the rear cell being connected through the aperture, the liquid pressure between the front cell and the rear cell being vacuum, the vacuum being used to make the sample of bacteria to be counted to pass from the front cell through the aperture into the rear cell, one of the electrodes being on each side of the aperture, and with the electrodes energized, there being a predetermined resistance between the two sides of the aperture;
detecting the presence of a pulse signal generated on both sides of the aperture due to a change in the resistance between the two sides of the aperture, wherein the pulse signal is used to indicate that a bacterium in the sample of bacteria to be counted passes through the aperture;
when a pulse signal generated on both sides of the aperture is detected, obtaining the number of bacteria in the sample of bacteria to be counted as determined on the basis of the pulse signal.

Optionally, the obtaining the number of bacteria in the sample of bacteria to be counted as determined on the basis of the pulse signal includes:
transmitting the pulse signal to a processing equipment, and obtaining the number of bacteria in the sample of bacteria to be counted sent by the processing equipment, wherein the number of bacteria in the sample of bacteria to be counted is obtained as determined on the basis of bacterial feature data indicated by the pulse signal; or
determining the number of bacteria in the sample of bacteria to be counted on the basis of bacterial feature data indicated by the pulse signal.

Optionally, the diameter of the aperture is a diameter within a first target diameter range, wherein the first target diameter range is used to allow only one bacterium to pass through the aperture at a time when bacteria in the sample of bacteria to be counted pass through the aperture; or
the diameter of the aperture is a diameter within a second target diameter range, wherein the second target diameter range is used to allow a plurality of bacteria to pass through the aperture at a time when bacteria in the sample of bacteria to be counted pass through the aperture.

There is one of the electrodes on each side of the aperture, a constant current source being formed by the electrodes on both sides of the aperture. However, the bacteria are not electrically conductive, thus generating a voltage pulse signal when passing through the aperture. Therefore, the number of bacteria in the bacteria sample to be counted can be determined by the described scheme based on the pulse signal. Optionally, the "pulse signal" can be a "voltage pulse signal".

Bacterial feature data indicated by the pulse signal includes: amplifying and gaining the pulse signal by a conditioning circuit, filtering noise by a low-pass filter, filtering over-limiting values by buffer limiting, and identifying signals with the bacterial feature data in the pulse signal by algorithms for pulse identification, slope identification, wave peak detection, wave trough detection and broadband detection among others.

Clogging of the aperture (aperture clogging) includes complete aperture clogging and incomplete aperture clogging, i.e., complete clogging of the aperture and incomplete clogging of the aperture.

In case of a complete aperture clogging, abnormal counts will be given, leading to an incorrect counting result, then a backwash module or a scorch module will be used to eliminate the aperture clogging; however, in case of an incomplete aperture clogging with data displayed, the test result will be directly affected. An incomplete aperture clogging can be distinguished by observing the counting time, i.e., there is a reference value for the observed counting time, and if the bacterial counting device is working properly with an unclogged aperture and the aspiration time of the sample of bacteria to be counted is be fixed, a prolonged counting time will indicate an incomplete aperture clogging in the detector of the bacterial counting device; and optionally, in another scheme, or in a case of aperture clogging, there is an algorithm to determine an over-limit number, thus determining that the data is inaccurate and that there is aperture clogging or external interference.

In a normal operating condition of the device, a fixed value of counting time is already set and hence the counting time is uniform, and in a normal operating condition, the voltage across the aperture is basically stable within a certain range, therefore, the occurrence of a rising voltage across the aperture or abnormal counts will indicate aperture clogging or impurity interference at the aperture. Many factors could lead to aperture clogging, and in most cases, it is because of an uneven mixing of a variety of bacteria, or infrequent cleaning of the aperture, which might lead to a buildup of non-counting substances and result in aperture clogging.

Optionally, there is another way to determine whether there is aperture clogging by the use of voltage interval, that is, the voltage is divided into 3 levels, i.e., normal, high or abnormal, respectively; in a case of a rising voltage, it is indicated that there is aperture clogging in the detector of the bacteria counting device, relatively high voltage indicating micro clogging of the aperture (i.e., incomplete aperture clogging), abnormal voltage indicating a complete aperture clogging, and normal voltage indicating a state without aperture clogging; in a case of a rising voltage of the aperture or abnormal counts, or abnormal baseline as determined, it is indicated that the aperture is clogged or interfered with impurities.

In a normal case: the middle liquid port of the rear cell is under vacuum, and there are 3 channels in the rear cell, the upper and lower channels being connected to a dilution buffer via a valve, the liquid passing inside being called uncontaminated liquid; a globe valve at the middle port then connecting to a pump, then the liquid being discharged as a waste liquid, an electrode also being located at the middle port (this electrode is an external electrode made of stainless steel, while the internal electrode made of platinum is located in the front cell), in a normal case the liquid in the middle being under vacuum to ensure that the liquid of the sample of bacteria to be counted can enter the rear cell from the front cell and that the counting is completed as the liquid of the sample passes the aperture, after enumeration of the sample of bacteria, the rear cell being cleaned by allowing a liquid to enter the upper and lower inlet ports of the rear cell and flow out from an outlet port on the other end of the rear cell, for example, the rear cell being cleaned by a liquid flowing into and out of the rear cell from the inlet and outlet ports of the rear cell, wherein for example, the fluid entering the rear cell is a dilution buffer and the fluid existing of the rear cell is a waste liquid, which may contain a sample and a dilution buffer, and the upper and lower channels being connected to each other as 1 divided into 2, 1 being the main channel leading to the dilution buffer, and 2 being connected to the upper and lower ports of the rear cell, respectively, and the middle channel being the channel with the electrodes.

In case of aperture clogging: the vacuum at the middle liquid port of the rear cell is switched off and one optional way is to start applying a positive pressure through a pressurized pump to generate pressure in the rear cell so as to backwash the aperture and eliminate the complete or incomplete clogging at the aperture. Another optional way is to feed liquid through the upper and lower liquid ports of the rear cell to generate pressure in the rear cell so as to backwash the aperture and eliminate the complete or incomplete clogging at the aperture.

Further, the counting cell module includes optionally:
a backwash module for backwashing in case of aperture clogging to eliminate the clogging of the aperture. Further, the vacuum on the liquid of the rear cell is switched off, and liquid is fed through two liquid ports of the rear cell to generate pressure in the rear cell so as to backwash the aperture and eliminate the aperture clogging. Alternatively, a positive pressure is applied through a pressurized pump to generate pressure in the rear cell so as to backwash the aperture and eliminate the aperture clogging.

Further, the counting cell module includes optionally:
a scorch module for scorching in a case of clogging at the aperture to eliminate the aperture clogging.

Further, the scorch module is used for providing a voltage higher than a predetermined voltage value through the electrodes to the aperture so as to melt the substances clogged in the aperture in a case of aperture clogging.

Once aperture clogging is detected by a computer connected to the bacterial counting device, i.e., the computer's alarm is set off or a message is prompted, a high-voltage scorching may be performed manually to eliminate the aperture clogging, i.e., by manually clicking the operation button on the computer (PC terminal) to start the high-voltage scorching circuit, i.e., while a DC voltage (relatively low-voltage) is applied for normal counting, a DC high-voltage is applied for scorching, the way of scorching being a rapid switching between a high and a low voltage, a high frequency being formed during the high-voltage scorching, and an arc discharge being generated on both sides of the aperture at the moments of current interruption, the generated electric sparks scorching the materials clogging in the aperture. Another optional way to eliminate aperture clogging by scorching is to use DC high voltage for scorching while a stable low-voltage is provided by a switch circuit for the normal counting, the high voltage during scorching heating the liquid to be detected to boiling so as to melt and eliminate the protein content to achieve an effect of scorching to eliminate aperture clogging.

Further, the predetermined voltage is between 90 and 110 V.

Further, the predetermined voltage is 110 V.

Further, the front cell is made of a plastic material.

Further, the front cell is made of a polyoxymethylene material.

Further, the rear cell is made of a plastic material.

Further, the rear cell is made of a polyoxymethylene material.

The good machining performance of plastic materials, especially polyoxymethylene materials, makes it easy to ensure required sizes of the front cell and the rear cell, and a more stable structure thereof.

The most outstanding beneficial effects of the method for measuring inhibition of bacteria by an antibacterial drug in embodiments of the present disclosure are as follows:
(1) A breakthrough in the reporting time. Reports can be sent within 1-2 hours of obtaining pure cultures.
(2) A breakthrough in practicality. However, the real advantage lies in that targeted antibiotics therapy can be achieved on the same day of obtaining a pure culture of bacteria together with a rapid identification of bacteria (completed within 1-2 hours) considering the daily workflow and schedules, which not only reduces the death rate in patients and healthcare costs, but also potentially slows the rise of bacterial drug-resistance.
(3) Mature and stable technology with reliable results.
(4) The principle is close to the international standardized protocol (broth dilution method for susceptibility test), which will have strong practicability if transformed into clinical practice.
(5) Low cost.
(6) Easy for automatization.

The bacterial counting device and technical scheme of the method thereof provided in the embodiments of the present disclosure have the following beneficial effects:
The embodiments of the present disclosure have realized an automated application of the device for enumeration of bacteria using the resistance counting method, have solved the problem of time consuming and inefficiency of current bacteria counting, and have achieved an effect of a fast and precise bacterial counting.

The improved aperture in the embodiments of the present disclosure ensures that bacteria can pass through the aperture one by one, thus preventing overlapping from affecting bacterial counting, and realizing accurate and efficient bacterial counting by the resistance counting method; the added functions of high-pressure backwashing and scorching can prevent aperture clogging, and in a case of aperture clogging, the high-pressure backwash design added in the rear cell can eliminate the complete or incomplete clogging of the aperture, and in a case of failed high-pressure backwashing, the scorching function can be used to eliminate the aperture clogging, that is, to ensure that under the condition of a smaller aperture, it is not easy to have the complete or incomplete clogging of the aperture.

A conditioning circuit for bacterial counting signals is specifically designed; a signal conditioning circuit is added to filter non-bacterial signals and accurately identify bacterial feature signals, resulting in reduced misjudgment.

### Brief Description of the Drawings

The drawings of the specifications, as part of the present disclosure, is provided for a further understanding of the present disclosure, and the schematic embodiments of the present disclosure and the description thereof are provided to explain the present disclosure, and do not constitute an undue limitation of the disclosure. In the drawings:

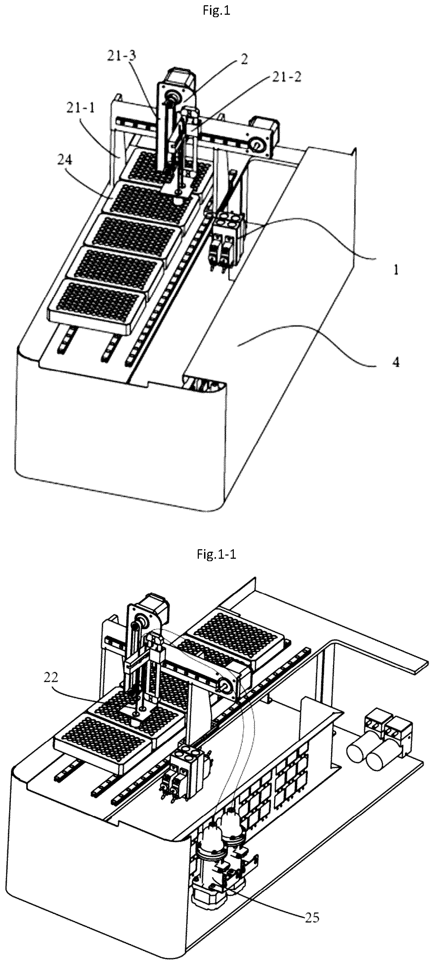

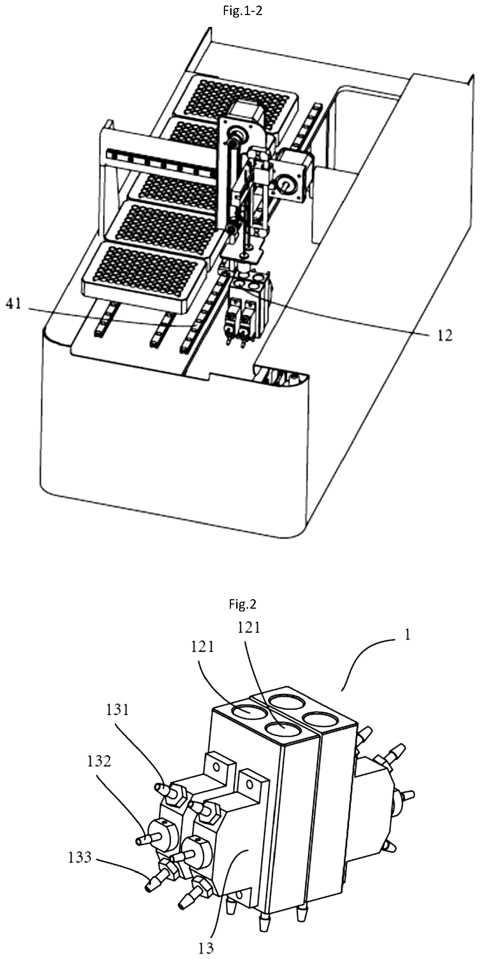

Fig. 1 schematically illustrates a schematic diagram of a complete bacterial counting device in accordance with an embodiment of the present disclosure;
Fig. 1-1 schematically illustrates a schematic diagram of a movable sample aspiration process of a complete bacterial counting device in accordance with an embodiment of the present disclosure;
Fig. 1-2 schematically illustrates a schematic diagram of a complete bacterial counting device dispensing an aspirated sample to a counting cell module in accordance with an embodiment of the present disclosure;
Fig. 2 schematically illustrates a structural schematic diagram of a counting cell module in accordance with an embodiment of the present disclosure;
Fig. 2-1 schematically illustrates a schematic diagram of a partially symmetrical cross-sectional structure of Fig. 2 in accordance with an embodiment of the present disclosure;
Fig. 2-2 schematically illustrates a schematic diagram of a cross-sectional structure of an aperture in accordance with an embodiment of the present disclosure;
Fig. 3 schematically illustrates a structural schematic diagram of a sampling module in accordance with an embodiment of the present disclosure;
Fig. 3-1 schematically illustrates a structural schematic cross-sectional diagram of a matching relationship of a sampling needle and a swab in accordance with an embodiment of the present disclosure;
Fig. 3-2 schematically illustrates a structural schematic diagram of a reagent plate in accordance with an embodiment of the present disclosure;
Fig. 4 schematically illustrates a schematic diagram of the working principle of a resistance counting according to an embodiment of the present disclosure;
Fig. 4-1 schematically illustrates a schematic diagram of the working principle of a liquid path diagram of a bacterial counting device in accordance with an embodiment of the present disclosure;
Fig. 5 schematically illustrates a schematic flowchart of a signal conditioning circuit in accordance with an embodiment of the present disclosure;
Fig. 6 is a schematic diagram of variation in bacteria counts at different time in an optional Escherichia coli broth culture in accordance with an example of the present disclosure;
Fig. 7 is a schematic diagram of variation in turbidity at different time in an optional Escherichia coli broth culture in accordance with an example of the present disclosure;
Fig. 8 is a schematic diagram of observed results of variation in turbidity and bacteria counts of an optional broth bacterial culture in accordance with an example of the present disclosure;
Fig. 9 is a schematic diagram of results of a growth experiment in Example 3 of the present disclosure;
Fig. 10 is a schematic diagram of results of a comparison between 2 h and 24 h in Example 3 of the present disclosure;
Fig. 11 is a schematic diagram of results of consistency rate of susceptibility in Example 3 of the present disclosure.

### Detailed Description of the Embodiments

It should be noted that the examples and the features in the examples of this disclosure can be combined with each other in the absence of conflict. The present disclosure will be in detail hereinafter with reference to the appended drawings and the examples.

The essence of susceptibility test (methods for measuring inhibition of bacteria by an antibacterial drug) of the present disclosure is to observe the effect of antibiotics on bacterial growth, metabolism and reproduction, and to infer the effectiveness of drug administration in the future according to the effect of the drug on bacterial growth, metabolism and reproduction (i.e., inhibition of bacteria) as observed in *in-vitro* test in combination with clinical and pharmacokinetics studies. In conventional methods, the killing effect of an antibiotic on bacteria is monitored by the changes in bacteria counts in a liquid or a solid medium, bacterial population being observed. Accurate quantitative monitoring of each individual bacterium, rather than the detection of variation trend in the sum counts of the bacterial population, can lead to an early and rapid detection of the effect of a drug on bacteria, thus leading to a major breakthrough for sure in the turn-around time of susceptibility test. However, there is a lack of an accurate, practical and automatable microscopic detection technique for individual bacterium in the prior art. Therefore, the technical schemes of the present disclosure are proposed creatively from another perspective to perform a rapid susceptibility test with the method of bacterial counting.

Specifically, one of the technical schemes of the present disclosure is a creative application of the most mature, reliable, rapid and economical resistance counting method (Coulter principle) for human blood cell counting to the method for measuring inhibition of bacteria by antibacterial drugs to achieve rapid susceptibility test. By adding different concentrations of an antibacterial drug during the growth of bacteria, it can be found that an antibacterial drug above a certain concentration inhibits the growth and reproduction of bacteria, thus determining the minimum inhibitory concentration. In conventional methods, the minimum inhibitory concentration is determined by changes in turbidity of the broth after bacterial growth, which takes a long time, usually 18 hours. In recent years some commercial companies have conducted optimization by using more sensitive nephelometers or adding redox indicators in an attempt to detect bacterial growth or inhibition at an early stage, however, it still takes 10 hours with these methods to deliver a report. Since antibacterial drugs can have an effect on bacteria in a short period of time, it is of practical importance to find a method or scheme to determine the effect of a drug on bacteria as quickly as possible, so that the susceptibility of bacteria to a drug can be determined in a very short period of time. The present disclosure can quantitatively count bacterial cells in a short period of time using resistance counting method, hence quickly determine the susceptibility to an antibacterial drug. Through analysis and comparison of changes in bacteria counts, the inhibitory effect of antibiotics on bacteria and hence antibacterial susceptibility can be quickly determined. This method is highly suitable for rapid susceptibility test with stable and reliable results.

A bacterial counting device is provided in an embodiment of the present disclosure. The bacterial counting device may be used for obtaining the current number of the bacteria in the mixture of bacteria and drug and the current number of the bacteria in the positive control in the method for measuring inhibition of bacteria by an antibacterial drug. This bacterial counting device measures the number of bacteria by using a resistance counting method. Firstly, it is required to invent a bacterial counting device for measuring the number of bacteria by using a resistance counting method, i.e. to design a sampling module, a counting cell module and a circuit control system, and to combine them to make a complete bacterial counting device suitable for measuring the number of bacteria by the resistance counting method. Further, since the aperture diameters of existing bacterial counting devices are suitable for measuring relatively large cells such as red blood cells and white blood cells, the technical scheme of the present disclosure is an improvement of the aperture diameter according to the size of bacteria specifically, i.e., the diameter of the aperture is adjusted so that only one bacterium or a plurality of bacteria of a sample of bacteria to be counted are allowed to pass through the aperture at a time when bacteria of the sample of bacteria to be counted pass through the aperture, and in an abnormal case of aperture clogging, a backwash module is used for backwashing to eliminate the clogging of the aperture, or a scorching module is used for scorching to eliminate the clogging of the aperture in a case of the aperture clogging.

### Exemplary Device

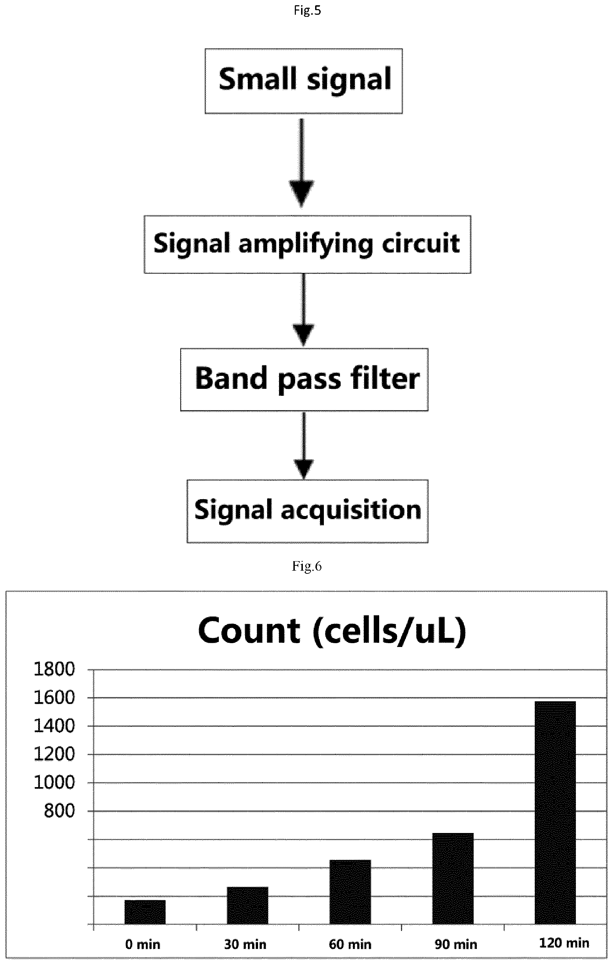

A schematic diagram of a complete bacterial counting device as described above is as shown in Fig. 1, the bacterial counting device comprising a counting cell module (1), a sampling module (2), a signal conditioning circuit (3) and a casing (4). The counting cell module (1) is fixedly connected to the sampling module (2). The signal conditioning circuit (3) is as shown in Fig. 5; the signal conditioning circuit (3) is located under the ropeway (41) as shown in Fig. 1-2, i.e., located in the bacterial counting device; the signal conditioning circuit (3) is connected to an internal electrode (141) and an external electrode (142) in the counting cell module (1); the signal conditioning circuit (3) includes a signal acquisition board, a master control board, etc. The casing (4) is located on the outside of the counting cell module (1), the sampling module (2) and the signal conditioning circuit (3), wherein the sampling module (2) includes a movement mechanism, and the sampling module (2) aspirates bacterial liquid to be tested into the counting cell module (1) through the movement mechanism, the counting cell module (1) being driven by the sampling module (2) to slide on the ropeway (41).

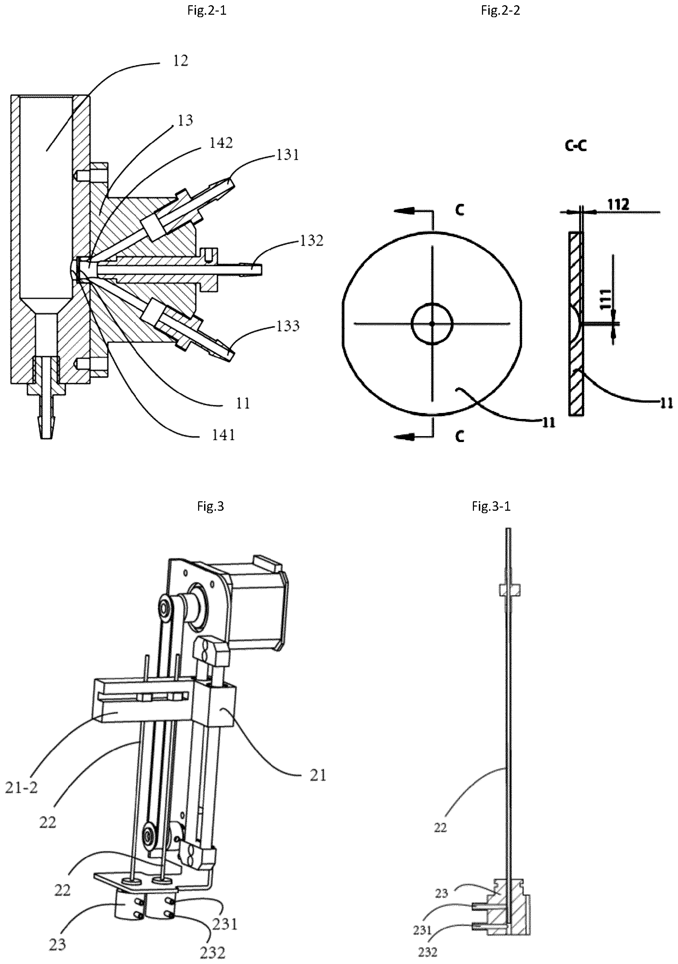

A schematic structural diagram of the counting cell module (1) is shown in Fig. 2, and a schematic structural diagram of the counting cell module (1) in partial section is shown in Fig. 2-1. The counting cell module (1) includes an aperture (11), a front cell (12), a rear cell (13), an internal electrode (141) and an external electrode (142) connecting the front cell and the rear cell, the aperture (11) being located between the front cell (12) and the rear cell (13), and the internal electrode (141) and the external electrode (142) being connected between the front cell (12) and the rear cell (13).

As shown in Fig. 2-1, the rear cell (13) includes an upper liquid port (131), a middle liquid port (132) and a lower liquid port (133), the liquid in the rear cell (13) being under vacuum, making all the bacterial liquid to be detected entering the front cell (12) to flow through the aperture (11) and to enter the rear cell (13) completely, wherein the most significant effect occurs when the middle liquid port (132) of the rear cell (13) is under vacuum; the upper liquid port (131) and the lower liquid port (133) in the rear cell (13) are two wash ports, a wire of the external electrode (142) being screwed on the metal of an outer wall of the middle liquid port (132), and optionally, the internal electrode (141) is platinum and is used for counting bacteria in the sample of bacteria to be counted. During testing, a sample of the liquid to be measured passes through the aperture, causing a change in resistance to be sensed by the electrodes in the front cell and the rear cell, thus generating a pulse signal in the circuit, and enumeration of bacteria is performed according to the number of pulses.

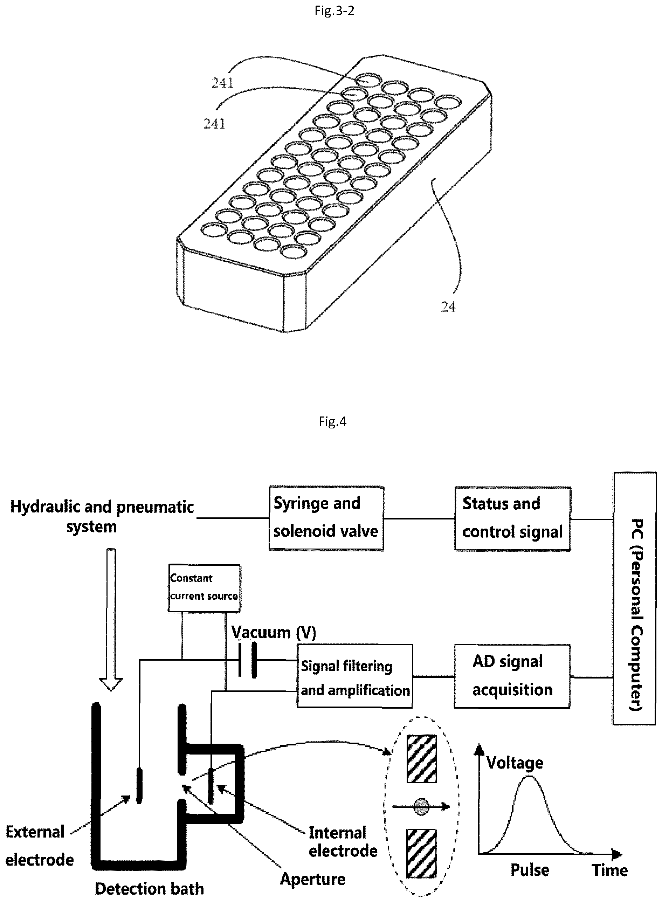

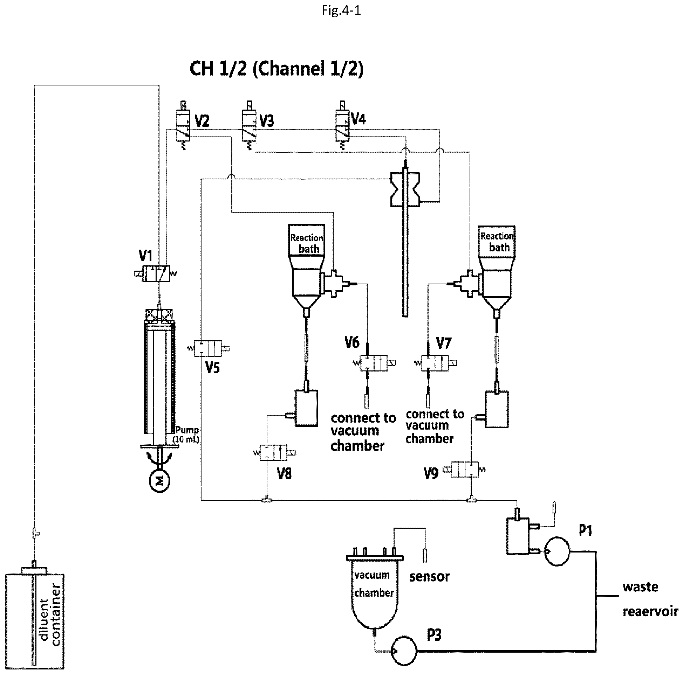

The strength of the signal of the internal electrode (141) and the external electrode (142) is measured by a receptor for bacterial counting. Since the dilution buffer is electrically conductive, a certain resistance is present at the aperture (11) when a certain voltage is applied between the two electrodes, and cells, which are not electrically conductive, will change the resistance of the aperture as they enter the aperture, thus generating a pulse signal in the circuit, the pulse signal then being processed and transmitted to the PC terminal for analysis, and the number, size and other parameters of the cells can be measured and analyzed according to the number of pulses and characteristics like pulse amplitude, etc. The working principle diagram is shown in Fig. 4, and the number of bacteria in the bacterial liquid to be measured is obtained through the resistance counting of the bacterial counting device and transmitted to a PC (computer) terminal.

A schematic diagram of the structure of the sampling module (2) is shown in Fig. 3. As shown in Fig. 1, Fig. 1-1 and Fig. 3, the sampling module (2) includes a mechanical arm that can move in three dimensions, a sampling needle (22), a swab (23), a reagent plate (24) and a plunger pump (25), etc. The movement mechanism of the sampling module (2) includes the mechanical arm and the sampling needle (22), the mechanical arm including a mechanical arm (21-1) for X-axis movement, a mechanical arm (21-2) for Y-axis movement and a mechanical arm (21-3) for Z-axis movement, one end of the sampling needle (22) passing through the swab (23). A schematic diagram of a partial sectional structure of the matching relationship of the sampling needle (22) and the swab (23) is as shown in Fig. 3-1. When cleaning is needed, water is fed by an inlet pipe (231) and then discharged by an outlet pipe (232). The other end of the sampling needle (22) is fixedly connected to the mechanical arm (21-2) and moves with the mechanical arm (21-2), thus realizing a targeted sampling function, i.e. the sampling needle (22) aspirates the bacterial liquid to be measured from the reagent plate (24). As shown in Fig. 1-1, the plunger pump (25) is connected to the sampling needle (22) and the plunger pump (25) controls the aspiration and discharge of the bacterial liquid to be measured by the sampling needle (22). Two of the sampling needles (22) are fixed to a rack of the 3-dimensional movement mechanical arm, and one of the swab (23) is attached to each of the sampling needles (22), or four of the sampling needles (22) can be used.

And a plurality of probes can not only be relatively stationary to each other, but can move independently.

As shown in Fig. 5 is a schematic structural diagram of the signal conditioning circuit (3), which is a signal conditioning circuit on a signal processing board and collects small signals, and then uploads the number of bacteria after amplification, filtering, signal acquisition, etc.

The working process of the sampling module of the bacterial counting device is schematically shown in Fig. 1, Fig. 1-1 and Fig. 1-2. When the bacterial counting device is working, the sampling module (2) moves rapidly to a designated position, and as controlled by the plunger pump (25), the sampling needle (22) performs an initial mixing of the bacterial liquid to be measured in the reagent plate (24) and aspirates a sample of the bacterial liquid to be measured, and then dispenses the bacterial liquid to be measured into the front cell (12) of the counting cell module (1) that moves along with the sampling module (2).

Optionally, the schematic structural diagram of the aperture 11 is shown in Fig. 2-2. Considering both the testing signal strength and bacterial counting time, the diameter (111, aperture diameter) of the aperture (11) is set in a range of 30 microns to 70 microns, preferably 40 microns to 60 microns, and the length (112) of the aperture (11) is 30 microns to 100 microns, preferably 40 microns to 70 microns. A diameter (111) of the aperture of 50 microns and a length (112) of the aperture of 50 microns is most suitable for bacteria measurement. In a case of clogging of the aperture (11), the high-pressure backwash design added to the rear cell (13) can eliminate the aperture clogging.

The test data of effects of the resistance counting for the same standard solution to be tested (roughly standard bacterial counts of 2500 cells/mL) with different diameters of the aperture using this design are as follows:
1) When the length of the aperture is set as 50 microns, the number of bacteria per milliliter (after calculation) measured with different diameters are compared as shown in Table 1.

**Table 1**

| No. / Aperture | 30µm | 40µm | 50µm | 60µm | 70µm |
|---|---|---|---|---|---|
| 1 | 1035 | 2132 | 2501 | 1142 | 936 |
| 2 | 1056 | 2200 | 2488 | 1132 | 921 |
| 3 | 1034 | 2150 | 2493 | 1135 | 902 |
| 4 | 1026 | 2140 | 2487 | 1136 | 910 |
| 5 | 1019 | 2157 | 2495 | 1156 | 930 |
| 6 | 1034 | 2169 | 2510 | 1158 | 940 |

The analysis demonstrates that if the diameter of the aperture is too small, aperture clogging will occur, leading to a decrease in the measured particle number. If the diameter of the aperture is too big, the number of particles passing by at the same time will increase, resulting in inaccurate counting and a decrease in the measured particle number. It can be seen from the above Table 1 that the optimal diameter (111) of the aperture is 50 microns, i.e., when the best test result data is obtained for resistance counting.

2) When the diameter of the aperture is set as 50 microns, the number of bacteria per milliliter (after calculation) measured with different apertures are compared as shown in Table 2.

**Table 2**

| No. / Length | 30µm | 40µm | 50µm | 60µm | 70µm | 80µm | 90µm | 100µm |
|---|---|---|---|---|---|---|---|---|
| 1 | 1700 | 1800 | 2501 | 1650 | 1025 | 951 | 850 | 725 |
| 2 | 1750 | 1800 | 2488 | 1645 | 1030 | 988 | 845 | 730 |
| 3 | 1705 | 1805 | 2493 | 1651 | 1050 | 993 | 851 | 750 |
| 4 | 1725 | 1795 | 2487 | 1648 | 1064 | 987 | 848 | 764 |
| 5 | 1736 | 1796 | 2495 | 1646 | 1036 | 995 | 846 | 736 |
| 6 | 1710 | 1810 | 2510 | 1652 | 1037 | 910 | 852 | 737 |

The analysis demonstrates that, under the condition of a fixed diameter of the aperture, a longer length of the aperture will increase the number of particles passing by at a same time, resulting in inaccurate counting and a decrease in the measured number of particles; if the length of the aperture is too short, because of the fast flow rate, many particles will not be detected, resulting in a decrease in the measured number of particles; as can be seen from the above Table 2, an optimal length of the aperture is 50 microns, i.e., when the best test effect data for resistance counting is obtained.

As can be seen from the above Table 2, it is optimal when the diameter (111) of the aperture is 50 microns and the length (112) of the aperture is 50 microns, i.e., with the best test effect data of the resistance counting. Although a diameter and length of the aperture in other ranges does not result in an effect as good as 50 microns, the counting can still be performed, and resulted inaccurate counting is relative, that is, for different bacterial liquid to be measured, a same counting standard leads to an accurate judgement of the trends of bacteria counts in the bacterial liquid, which indicates that other length and diameter specifications of the aperture can still be used to measure the magnitude of the bacterial counting.

The high-pressure backwash design specifically is as follows: switch off the vacuum on the middle liquid port (132) of the rear cell (13) so that liquid is fed in through the upper liquid port (131) and lower liquid port (133) of the rear cell (13), generating pressure in the rear cell (13) to backwash the aperture (11) and eliminate the complete or incomplete clogging of the aperture (11). In case the high-pressure fails, a scorching function can be selected to eliminate aperture clogging, that is, it is not easy to have the complete or incomplete aperture clogging while ensuring a smaller aperture.

Optionally, as shown in Fig. 2, the front cell (12) is an integrated quad-channel structure using a polyoxymethylene material or other plastic materials, the distance between the two channel ports (121) of the front cell (12) being 18 mm, and the volume of the internal liquid of the front cell (12) is greater than 2.5 mL. The employment of a polyoxymethylene material or other plastic materials make it easy to ensure accurate sizes of the front cell (12) and rear cell (13) and a more stable structure thereof.

As an example, bacteria might cause clogging of the aperture while passing through the aperture, resulting in inaccurate counting of bacteria. In order to solve the problem of inaccurate counting of bacteria due to clogging generated in the aperture, a detection scheme and an elimination scheme for clogging in the aperture are also provided in an example of the present disclosure. As shown in Fig. 1-1 is a diagram of an optional complete device, wherein a plunger pump is provided next to the bacteria counting device for high-pressure backwashing to eliminate the clogged aperture, or/and a high-frequency counting voltage is applied to the electrodes at both ends of the aperture for high-voltage scorching to eliminate the clogged aperture. See the exemplary method for detailed operation.

### Exemplary method

Optionally, a platinum electrode is located on each side of a laser-formed aperture. Since the dilution buffer is electrically conductive, a certain resistance is present at the aperture when a certain voltage is applied between the two electrodes. But the cells are not electrically conductive and they will change the resistance of the aperture as they enter the orifice, thus generating a pulse signal in the circuit, the pulse signal then being processed and transmitted to a PC terminal for analysis, and the number, size and other parameters of the cells can be measured and analyzed according to the number of pulses and the characteristics like pulse amplitude, etc.

A signal conditioning circuit and an acquisition algorithm are specifically designed for bacteria counting, the effective signals being kept intact through signal amplification, and the effective signal being adjusted to an amplification which is most conducive to algorithm recognition by adjusting the gain; low-pass filtering is used to filter out the high-frequency noise, and over-limiting amplitude is filtered out by buffer limiting. The number of bacteria is obtained from the pulse signal by accurately identifying the bacterial feature signals through algorithms such as pulse recognition, slope recognition, crest detection, trough detection, and broadband detection.

When the pulse signal includes a set of pulse signals of a first type, the circuit control system or processing equipment determines the number of the set of pulse signals of a first type as a first count, wherein each pulse signal of the set of pulse signals of a first type is a pulse signal triggered by one of the bacteria when passing through the aperture; when the pulse signal includes a set of pulse signals of a second type, the circuit control system or processing equipment determines the product between the number of the set of pulse signals of a second type and a predetermined number as a second count, wherein each pulse signal of the set of pulse signals of a second type is a pulse signal triggered by the predetermined number of the bacteria when simultaneously passing through the aperture.

When the pulse signal includes only a set of pulse signals of the first type, the number of bacteria in the sample to be counted is determined as the first count; in the case where the pulse signal includes only a set of pulse signals of the second type, the number of bacteria in the sample to be counted is determined as the second count; when the pulse signal includes a set of pulse signals of the first type and a set of pulse signals of the second type, the number of bacteria in the sample of bacteria to be counted is determined as the sum of the first count and the second count.

As an example, the circuit control system or processing equipment in an example of the present disclosure may determine whether the pulse signal includes a set of pulse signals of the second type through the following steps:
In a case of only one bacterium passing, it is accurate counting; in a case of two or three bacteria passing through the aperture simultaneously, the generated pulse signal is a second type pulse signal, and it is recorded as effective counting if the second type pulse signal is within the error range of the first type pulse signal, otherwise an error is reported to recount or the count result is converted by the error value.

As an example, bacteria might cause clogging of the aperture while passing through the aperture, resulting in inaccurate counting of bacteria. In order to solve the problem of inaccurate counting caused by clogging of the aperture, a detection scheme and an elimination scheme are further provided in the example of the present disclosure.

As an exemplary detection scheme for aperture clogging, the example of the present disclosure further includes: when the voltage between the front cell and the rear cell is detected as exceeding a predetermined threshold, it is determined that there is clogging in the aperture, wherein the front cell is the anode and the rear cell is the cathode, and the more serious the aperture clogging is, the greater the resistance between both sides of the aperture is, and hence the greater the voltage between the front cell and the rear cell is; the predetermined threshold can be set according to different measurement requirements (such as different measurement accuracy) for bacteria counts.

As an exemplary scheme for elimination of aperture clogging, the scheme of backwashing for elimination of aperture clogging in the example of the present disclosure further includes:
As an exemplary scheme for elimination of aperture clogging, the scheme of scorching for elimination of the aperture clogging in the example of the present disclosure further includes: high-pressure blackwashing and high-voltage scorching.

In case of aperture clogging, a liquid is backwashed under positive pressure from the middle washing port directly-facing the position of the aperture in the middle part and discharged from the upper and lower washing ports; under a combined control of the plunger pump and the valve, a positive pressure is applied to the rear cell through the upper and lower liquid ports, the waste liquid being discharged from the waste liquid port of the front cell, the waste liquid being extracted through a combination of a solenoid valve and a waste liquid pump.

The process of scorching is performed under 110 V with a high-frequency counting voltage being applied to the electrodes at both ends of the aperture; for normal counting, the counting voltage is a continuously provided DC voltage, but for high-voltage scorching, it is designed to power on and off at short intervals, forming a high frequency, and at the moment of power on and power off, an arc discharge is generated between the two electrodes, the aperture being the emitting point of an electric spark, so that the protein and debris are easily removed. In some instruments it is designed to have a separate AC power supply, wherein the scorching can be controlled through a relay or a silicon controlled rectifier (SCR) at the front end of the electrode wire. Optionally, scorching to eliminate aperture clogging can also be achieved by melting proteins through autoclaving heating.

Optional is a multi-probe design with a novel probe structure, 2 probes moving at the same time to achieve the effect of 4 probes, thus saving costs; this design is applied to bacterial counting with 2 stainless steel sampling probes being driven by a 3-dimensional movement mechanical arm, as shown in Fig. 3, the distance between two of the sampling needles (22) being 18 mm, to aspirate samples at the target plate and discharge the liquid to be measured into two of the counting cells, 4 of which in total, and two counting cell channel beginning to work at this time; then the 3-dimensional movement mechanical arm drives the two stainless steel sampling probes to aspirate samples at the target plate again and discharge the liquid to be measured into the other two counting cells, then these two counting cell channel also begin to work; once a channel detection is completed, the samples will be aspirated again and the same actions will be repeated. Simultaneous motion of 2 probes (the sampling needles (22)) and multi-channel detection not only save waiting time but also save the cost of additional probe assemblies.

Optionally, the 3-D arm moves with the counting cell to shorten the time between sample aspiration and sample delivery.

The 3-D arm moves together with the counting cell, being relatively stationary. When detection is required, a sample is directly placed into a counting cell in close proximity by the 3-D arm moving in X, Y and Z axes, while no excessive 3-D movements are required.

Optionally, as shown in Fig. 3, the sampling needles (22) are spaced 18 mm apart; as shown in the schematic structural diagram of the reagent plate (24) in Fig. 3-2, the interval of two test holes (24I) of the reagent plate (24) is 9 mm, hence the two sampling needles (22) are spaced exactly twice the interval of the test holes of the reagent plate, thus resulting in less travel so as to save time while avoiding long movement travel, increased running time and reduced efficiency caused by a too large interval.

Optionally, as shown in Fig. 4, once the liquid is discharged by the sampling needles into the front cell, the liquid in the front cell is taken to the rear cell under the action of vacuum and so the liquid passes through the aperture; when there are bacteria passing through the aperture, a pulse signal is generated (a constant current source is provided, and bacteria passing indicates a change in resistance and in turn a change in voltage is generated), the circuit is filtered, and the signal is amplified and then filtered, after that, the signal reaches the single-chip microcomputer, where AD acquisition is carried out, moreover, the single-chip microcomputer has a pulse recognition algorithm, the processed data will be uploaded to a PC software. During signal processing: AD acquisition takes up roughly 10M magnitude data, and then processed into data of a number of K by the algorithm, this "a number of K" being the total counts and histogram information. Then the data is uploaded to a PC computer.

Optionally, the work flow diagram of the liquid flow of the bacteria counting device in the working process is shown in the liquid path diagram Fig. 4-1. The bacteria counting device is a bacteria counting device with a liquid path of quad-channel counting. Since the liquid path of each channel is consistent, two of the channels, i.e., channel 1 and channel 2 (CH1 and CH2) are taken as an example to elaborate its working principle: as shown in Fig. 4-1, the liquid path diagram, before adding samples to count, the counting cell module (1) will be cleaned first each time before counting; dilution buffer (bacteria reagent to be measured) is aspirated by the solenoid valve (V1) in combination with the 10 mL pump, then the liquid is injected into the front cell (12) through the cooperation of the solenoid valve (V1), the solenoid valve (V2), the solenoid valve (V3), the solenoid valve (V4) and the pump, then a positive pressure is applied to the liquid path through the cooperation of the 10 mL pump and the solenoid valve (V1), the solenoid valve (V2) and the solenoid valve (V3) to backwash the aperture (11), then the waste liquid in the front cell (12) is discharged completely by the solenoid valve (V8), the solenoid valve (V9) and the pump (P1), while the waste liquid in the rear cell (13) is discharged by the pump (P3), the solenoid valve (V6) and the solenoid valve (V7). Wherein the process of sample dispensing and counting is as follows: dispensing the liquid into the front cell (12), then diluting by adding dilution buffer through a combination of a solenoid valve and a pump, lifting the swab (23). The cleaning processing for the swab (23) is as follows: lifting the sampling needles (22), the bottom surface of the sampling needle (22) being wrapped in the swab (23), the dilution buffer that washes the outer walls of the sampling needles (22) from the channel of the solenoid valve (V4) will be discharged into the waste reservoir by a cooperation of the solenoid valve (V5) and the pump (P1), then the dilution buffer that washes the inner walls of the sampling needles (22) from the channel of the solenoid valve (V4) will be discharged into the waste reservoir by a cooperation of the solenoid valve (V5) and the pump (P1). The solenoid valve (V4) is a tee valve with one inlet and two outlets; at least one of the two outlets (say outlet 1 and outlet 2) is open to the inlet at a certain moment so as to control the dilution buffer to clean the inner walls and the outer walls of the sampling needles (22); the needles will take samples to be measured after cleaning. Counting for a certain amount of time in the two channels which samples are dispensed into first is performed through vacuum generated by the solenoid valve (V6) and pump (P3), and once the predetermined time is reached, the front cell (12) and the rear bath (13) will be cleaned through a combination of the solenoid valve and pump thereof, wait for the next sample dispensing.

It should be noted that although a plurality of units/modules or subunits/submodules are mentioned in the detailed description above, the division is only exemplary but not compulsory. In fact, according to the embodiments of the present disclosure, the features and functions of two or more of the units/modules as above can be specified in a single unit/module. Alternatively, the features and functions of a single unit/module as above can be divided and specified in several units/modules.

Furthermore, although the operations of the method of the present disclosure are in the accompanying drawings in a particular order, it is not required or implied that the operations must be performed in that particular order or that all of the operations shown must be performed to achieve the desired results. Additionally or alternatively, certain steps may be omitted, multiple steps may be combined into one step for execution, and/or one step may be broken down into multiple steps for execution.

Beneficial effects of the method for measuring inhibition of bacteria by an antibacterial drug will be further hereinafter with reference to examples.

### Example 1

Objective: to find the susceptibility indicators for bacterial changes in broth cultures, to determine the theoretical basis for rapid drug susceptibility testing, and to establish a method for bacterial drug susceptibility testing.

### Materials and Methods

### 1. Preparation of bacterial strains

Three standard strains (Escherichia coli ATCC 25922, Staphylococcus aureus ATCC 25923 and Pseudomonas aeruginosa ATCC 27853) were transferred to be used later and incubated at 37°C for 18 hours.

### 2. Preparation of broth

Prepare the bacterial strain and triturate on the wall of a vial with AST (antibiotics susceptibility testing) broth to mix evenly, close the cap and measure the turbidity with a nephelometer (BD PhoenixSpec Nephelometer), the turbidity being 0.5 McFarland units, and set aside to be used later. Inoculate the bacteria to be tested at an inoculum concentration specified in the CLSI (American Clinical and Laboratory Standards Institute) method for broth dilution drug susceptibility testing, and incubate in an incubator at 37°C (degrees Celsius).

### 3. Bacterial counting

Incubate in an incubator at 37°C, perform bacterial counting at 0 min, 10 min, 30 min, 60 min, 90 min and 120 min by the resistance counting method (OMEC (Zhuhai) RC-3000 resistance (Coulter) particle counter). Measure twice, take the average value and record the data.

### 4. Measurement of turbidity

Incubate in an incubator at 37°C, measure the turbidity twice at 0 min, 10 min, 30 min, 60 min, 90 min and 120 min with a nephelometer (BD PhoenixSpecNephelomter), take the average and record the data. Results are as follows in Table 3:

**Table 3 the observed results of turbidity and bacterial count variation in a broth bacterial culture**

| | **ATCC 25922** | | **ATCC 25923** | | **ATCC 27853** | |
|---|---|---|---|---|---|---|
| | Escherichia coli | | Staphylococcus aureus | | Pseudomonas aeruginosa | |
| | Turbidity | Count (cells/µL) | Turbidity | Count (cells/µL) | Turbidity | Count (cells/µL) |
| 0 min | 0.04 | 172 | 0.04 | 211 | 0.04 | 141 |
| 30 min | 0.05 | 265 | 0.04 | 254 | 0.04 | 246 |
| 60 min | 0.05 | 456 | 0.05 | 376 | 0.04 | 338 |
| 90 min | 0.06 | 647 | 0.06 | 697 | 0.05 | 668 |
| 120 min | 0.08 | 1577 | 0.06 | 963 | 0.06 | 1013 |

The changes in the number of bacteria at different time in an Escherichia coli broth culture is as shown in Fig. 6;

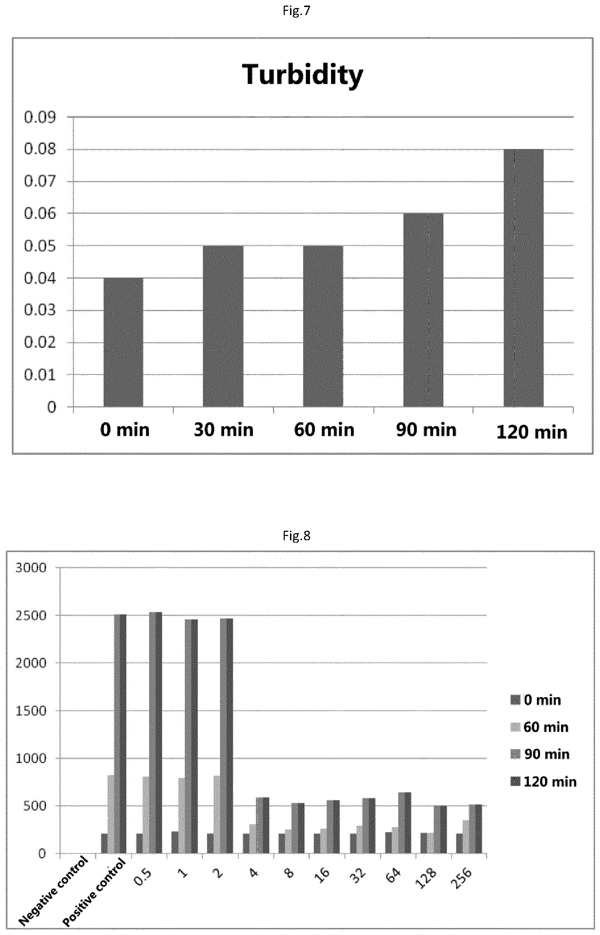

The changes in turbidity at different time in an Escherichia coli broth culture is as shown in Fig. 7.

### Conclusions

1. In the case of broth culture for bacteria, great difference in the observed bacterial changes is demonstrated between different methods, and the currently commonly used turbidimetric methods for population growth of bacteria show a poor sensitivity and cannot determine the difference within 120 minutes.
2. The bacterial counting method can determine significant difference within 30 minutes of broth cultivation, which demonstrates the feasibility of a bacterial drug susceptibility testing method.

### Example 2

A method for measuring inhibition of Escherichia coli ATCC 25922 by ampicillin (by the resistance counting method).

### Materials and Methods

### 1. Preparation of bacterial strains

The standard strain ATCC 25922 Escherichia coli was transferred for use later and incubated at 37°C for 18 hours.

### 2. Preparation of broth

Ten of the drug susceptibility testing tubes (or cups) contained the required antibiotics of concentrations via doubling dilution (refer to American CLSI standards for concentrations for different drugs); the eleventh tube without antibiotics is used as a positive control (PC); the twelfth tube without bacterial suspension is used as a negative control (NC).

Prepare the bacterial strain and triturate on the wall of a vial with Mueller Hinton broth (MH for antibiotics susceptibility testing) to mix evenly, close the cap and measure the turbidity with a nephelometer (BD PhoenixSpec Nephelometer), the turbidity being 0.5 McFarland units, and set aside to be used later.

Inoculate the bacteria to be tested at an inoculum concentration specified in the CLSI (American Clinical and Laboratory Standards Institute) method for broth dilution drug susceptibility testing, and incubate in an incubator at 37°C.

Preparation of bacterial suspension: pick a spare colony to prepare bacterial suspension, the concentration of the bacterial suspension being 0.5 McFarland units. Add the colony suspension to (MH) broths containing different concentrations of various antibiotics and the content of bacteria in each tube after inoculation is 1X10^4 cfu/mL to 5X10^7 cfu/mL, optimally 5X10^6 cfu/mL (colony forming units/mL), i.e. optimally 5X10^6 colony forming units per mL.

### 3. resistance counting method (Coulter principle)

Incubate in an incubator at 37°C, perform bacterial counting at 0 min, 10 min, 30 min, 60 min, 90 min and 120 min respectively by the resistance counting method (OMEC (Zhuhai) RC-3000 resistance (Coulter) particle counter). Measure twice, take the average value and record the data.

The change in bacterial counts in the rapid susceptibility test of Escherichia coli ATCC 25922 to ampicillin is shown in Table 4 below.

**Table 4**

| | Negative control | Positive control | 0.5 | 1 | 2 | 4 | 8 | 16 | 32 | 64 | 128 | 256 |
|---|---|---|---|---|---|---|---|---|---|---|---|---|
| 0 min | 0 | 204 | 210 | 230 | 208 | 206 | 204 | 208 | 210 | 220 | 212 | 207 |
| 60 min | 0 | 820 | 807 | 789 | 811 | 304 | 250 | 264 | 290 | 278 | 214 | 350 |
| 90 min | 0 | 1502 | 1540 | 1469 | 1511 | 432 | 502 | 500 | 491 | 489 | 367 | 460 |
| 120 min | 0 | 2513 | 2534 | 2456 | 2463 | 590 | 524 | 560 | 578 | 641 | 498 | 512 |

The observed results of turbidity and changes in bacterial counts in the broth culture are shown in Fig. 8, wherein the horizontal axis indicates the concentration of antibiotic drugs, corresponding to the values in the first row in Table 4 in the appendix, the unit being µg/mL; the vertical axis indicates the bacterial counts, the unit being cells/µL.

There were 12 tubes for testing, the eleventh tube being the positive control (PC), the twelfth tube being the negative control (NC) and the other 10 tubes being the test tubes. At each detection time point, measurements were carried out for all 12 tubes.

Fig. 8 shows the results of the drug susceptibility testing by use of resistance method, that is, testing of inhibition of Escherichia coli ATCC 25922 by ampicillin in the present example, and the result demonstrated a minimum inhibitory concentration (MIC) of 4 µg/mL.

In addition, the bacteria in this example were also subjected to three other methods of drug susceptibility testing, namely, measuring the inhibition of Escherichia coli ATCC 25922 in this example by ampicillin according to the operating manual of the VITEK microbial identification susceptibility system from Merieux, France, with a minimum inhibitory concentration of 4 µg/mL (micrograms per milliliter); measuring the inhibition of Escherichia coli ATCC 25922 in this example by ampicillin according to the Etest method, as detailed in the operating manual of the Etest kit from Thermo Fisher, USA, with a minimum inhibitory concentration of 2 µg/mL; and measuring the inhibition of Escherichia coli ATCC 25922 in this example by ampicillin according to broth dilution drug susceptibility testing method as specified in the broth dilution susceptibility standards by the American Clinical and Laboratory Standards Institute, with a minimum inhibitory concentration of 4 µg/mL. It can be seen that their results are all consistent and sensitive.

### Conclusions

1. The minimum inhibitory concentration (MIC) was determined within 60 minutes.
2. The minimum inhibitory concentration (MIC) was determined within 60 minutes in the example of the present disclosure, which is consistent with the results of the VITEK (Merieux drug susceptibility testing method) method, the Etest method and the broth dilution drug susceptibility testing, i.e., it is demonstrated again the feasibility of the bacterial drug susceptibility testing method of the present disclosure, which shows significant beneficial effects of rapidly obtaining bacterial susceptibility results.

As can be seen from Examples 1 and 2, the inhibition results of the method for measuring bacterial inhibition of antibacterial drugs of the present disclosure were analyzed in comparison with the results of conventional methods: the results were compared with the results of VITEK (Merieux drug susceptibility testing method), Etest and BMD (broth dilution method), and the comparison criteria were determined according to the FDA (U.S. Food and Drug Administration) regulations.

The points for result determination of drug susceptibility testing is when the observed cell count reduces 20%, 40%, 60% or 80% compared with the positive control, and the results are compared with conditional methods for consistency. The conclusion is that when the cell count was reduced by 40% to 60% or more, the accuracy reached 100%, wherein it was optimal when the cell count was reduced by 60% or more. The time to reach a cell count reduction of 40% to 60% varied among different bacteria, and the testing can be completed within 90 to 120 minutes for common clinical bacteria, and 90 minutes for most common clinical bacteria.

### Example 3

### 1. Growth experiment

### 1.1 Experiment objectives

In accordance with the requirements of the CLSI standards, 6 common strains which are commonly used in clinical field (ATCC 29212, ATCC 29213, ATCC 27853, ATCC25922, Klebsiella Pneumoniae ATCC 700603 and Acinetobacter baumannii) were inoculated, and their growth trends were recorded to determine if there was a possibility to determine their growth.

### 1.2 Experiment method

1.2.1 Negative control: testing for uninoculated media with a resistance bacterial counter, and recording the particle counts;
1.2.2 Preparation of bacterial strains: In accordance with the requirements of the CLSI standards, pick a fresh strain within 24h with a turbidity of 0.5 McFarland units, and take 100 µL and dispense to a medium of 10 mL;
1.2.3 Record the results at 0h/ 0.5h/ 1h/ 1.5h, respectively, and analyze the results after removal of the negative background.

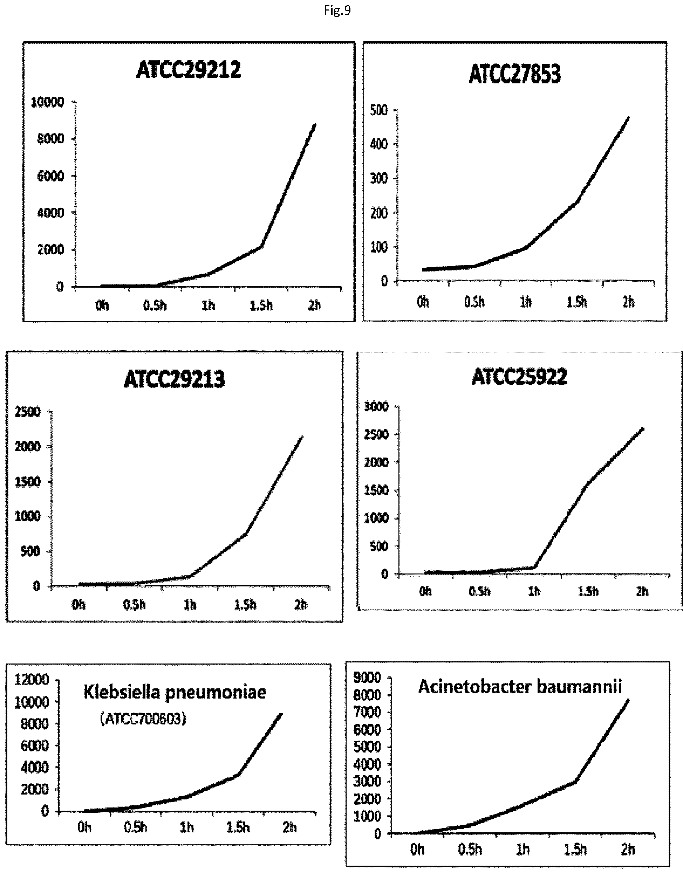

2.3 Results are shown in Fig. 9. It can be seen from the growth experiment that there was already a significant change in the bacteria at 2h, which was clearly captured by the resistance counting method, thus demonstrating the feasibility of resistance counting in terms of observational detection.

2. Result of comparison of susceptibility at 2h and 24h (the feasibility of the resistance method for bacterial susceptibility was determined by comparing the results of susceptibility during the two time periods).

### 2.1 Experiment objectives

A group of Class A drugs were selected for each bacteria strain commonly used in clinical practice to carry out the result comparison and examine the consistency between results of 2h and 24h. See Table 5 for the bacterial strains and each corresponding antibiotic.

**Table 5**

| Name of bacterial strain | Antibiotics |
|---|---|
| Pseudomonas aeruginosa (ATCC27853) | Ceftazidime |
| Staphylococcus aureus (ATCC29213) | Erythromycin |
| Enterococcus faecalis (ATCC29212) | Penicillin |
| Klebsiella pneumoniae | Gentamicin |
| Acinetobacter baumannii | Meropenem |

### 2.2 Experiment method

2.2.1 Preparation of bacterial strains: In accordance with the requirements of the CLSI standards, pick each fresh strains within 24h with a turbidity of 0.5 McFarland units in the column of strain name in Table 5;
2.2.2 Inoculation: Take 100 µL of each bacterial suspension with a turbidity of 0.5 McFarland units and dispense to a 48-well plate with pre-configured gradient antibiotics, 500 µL dispensed in each well;
2.2.3 Incubation and detection: Incubate for 2h at 37°C, and measure with a resistance bacterial counter. Adjust the instrument to its best sensitivity and then count bacteria. Record the 24h routine susceptibility results for comparative analysis.

### 2.3. Analysis of experiment results

**Table 6**

| Name of bacterial strain | Antibiotics | 2hMIC | 24hMIC | Notes |
|---|---|---|---|---|
| Pseudomonas aeruginosa (ATCC27853) | Ceftazidime | 4 | 2 | Difference of one gradient, acceptable |
| Staphylococcus aureus (ATCC29213) | Erythromycin | 0.5 | 0.25 | Difference of one gradient, acceptable |
| Enterococcus faecalis (ATCC29212) | Penicillin | 1 | 2 | Difference of one gradient, acceptable |
| Klebsiella pneumoniae | Gentamicin | 4 | 8 | Difference of one gradient, acceptable |
| Acinetobacter baumannii | Meropenem | 0.12 | 0.12 | No difference |

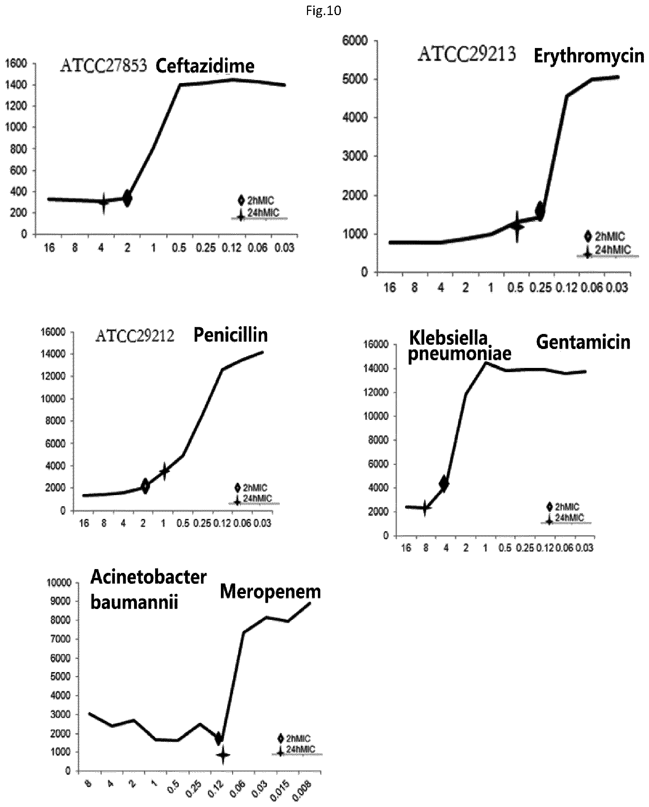

It can be seen from the results in Fig. 10 and Table 6 that difference in an acceptable range was exhibited of the MIC obtained by resistance counting method (60% of the positive count value as the threshold to determine the MIC) of the preferred drugs as required by CLSI for the five clinically common bacterial strains selected for antibiotics susceptibility testing, as compared with the conventional drug susceptibility testing method by visual observation after 24 h, thus proving the feasibility of the resistance counting method for bacterial drug susceptibility testing.

### 3. Susceptibility consistency rate

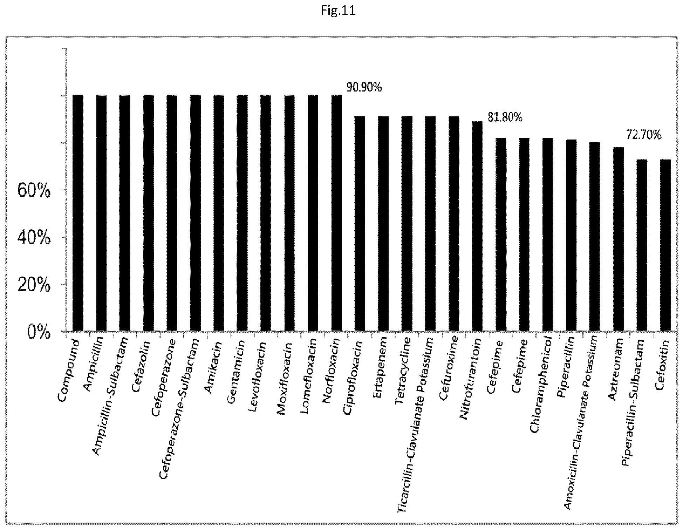

Eleven clinically common enterobacteria strains (2 strains of Escherichia coli, Morganella morganii, Shigella Castellani, Klebsiella aerogenes, Citrobacter freundii, 2 stains of Klebsiella pneumoniae, Proteus mirabilis, Enterobacter cloacae, Salmonella spp.) were selected; 96-well enterobacteria susceptibility reagent plates produced by Scenker Biological Technology Co., Ltd were used for inoculation according to CLSI requirements; the bacteria were incubated at 37°C for 2h before inoculation on two reagent plates, one reagent plate being used for a 2h recording of bacterial counting results and comparison with the results of the positive control. Preliminarily positive result values of not less than 60% of the result values are taken as a positive value for results recording. The other reagent plate was used for recording experiment results at 24h and for determining susceptibility consistency rate between 2h and 24h. As shown in Fig. 11, the results indicated that the drug susceptibility testing of the 11 clinically common Enterobacteriaceae species to the commonly used clinical antibiotics by resistance counting method exhibited a high consistency rate compared with the conventional CLSI results.

### Example 4

Results of drug susceptibility testing for live bacteria and consistency with conventional methods

### 1.1 Experiment objectives

A group of Class A drugs were selected for each bacteria strain commonly used in clinical practice to carry out the result comparison and examine the consistency between results of 2h and 24h. The growth trends were recorded to determine if there was a possibility to determine their growth at 2h. See Table 7 for the bacterial strains and each corresponding antibiotic.

**Table 7**

| Name of bacterial strain | Antibiotics |
|---|---|
| Pseudomonas aeruginosa (ATCC27853) | Ceftazidime |
| Staphylococcus aureus (ATCC29213) | Erythromycin |
| Enterococcus faecalis (ATCC29212) | Penicillin |
| Klebsiella pneumoniae | Gentamicin |
| Acinetobacter baumannii | Meropenem |

### 1.2 Experiment method

1.2.1 Preparation of bacterial strains: in accordance with the requirements of the CLSI standards, pick each fresh strains within 24h with a turbidity of 0.5 McFarland units in the column of strain name in Table 7.

1.2.2 Inoculation: take 100 µL of each bacterial suspension with a turbidity of 0.5 McFarland units and dispense to a 48-well plate with pre-configured gradient antibiotics, 500 µL dispensed in each well.

1.2.3 Incubation and detection: incubate for 2h at 37°C, and perform live bacteria counting. Record the 24h routine susceptibility results for comparative analysis.

### 1.3 Analysis of experiment results

### 1.3.1 Experiment results

Drug susceptibility testing results of Pseudomonas aeruginosa to ceftazidime:
Results of the rapid drug susceptibility testing of living cells: MIC = 2; results of conventional drug susceptibility testing: MIC = 2.

See Table 8 for detailed data of the rapid drug susceptibility testing of living cells.

Table 8 (The values in the first row are the concentrations of the antibiotic drug, the unit being µg/mL; values in the second row are bacterial counts, the unit being cells/µL.)

| | 16 | 8 | 4 | 2 | 1 | 0.5 | 0.25 | 0.12 | 0.06 | 0.03 | Positive control |
|---|---|---|---|---|---|---|---|---|---|---|---|
| Living cell counts | 209 | 202 | 297 | 308 | 1090 | 1235 | 1480 | 1513 | 1478 | 1509 | 1498 |

Drug susceptibility testing results of Staphylococcus aureus to erythromycin:
Results of the rapid drug susceptibility testing of living cells: MIC = 0.25; results of conventional drug susceptibility testing: MIC = 0.25.

See Table 9 for detailed data of the rapid drug susceptibility testing of living cells.

Table 9 (The values in the first row are the concentrations of the antibiotic drug, the unit being µg/mL; values in the second row are bacterial counts, the unit being cells/µL.)

| | 16 | 8 | 4 | 2 | 1 | 0.5 | 0.25 | 0.12 | 0.06 | 0.03 | Positive control |
|---|---|---|---|---|---|---|---|---|---|---|---|
| Living cell counts | 457 | 446 | 460 | 498 | 598 | 609 | 889 | 3980 | 5078 | 5107 | 4897 |

Drug susceptibility testing results of Enterococcus faecalis to Penicillin:
Results of the rapid drug susceptibility testing of living cells: MIC = 1; results of conventional drug susceptibility testing: MIC = 2.

See Table 10 for detailed data of the rapid drug susceptibility testing of living cells.

Table 10 (The values in the first row are the concentrations of the antibiotic drug, the unit being µg/mL; values in the second row are bacterial counts, the unit being cells/µL.)

| | 16 | 8 | 4 | 2 | 1 | 0.5 | 0.25 | 0.12 | 0.06 | 0.03 | Positive control |
|---|---|---|---|---|---|---|---|---|---|---|---|
| Living cell counts | 659 | 683 | 798 | 1076 | 3085 | 11105 | 12088 | 12958 | 13679 | 13789 | 13900 |

Drug susceptibility testing results of Klebsiella Pneumoniae to Gentamicin:
Results of the rapid drug susceptibility testing of living cells: MIC = 8; results of conventional drug susceptibility testing: MIC = 8.

See Table 11 for detailed data of the rapid drug susceptibility testing of living cells.

Table 11 (The values in the first row are the concentrations of the antibiotic drug, the unit being µg/mL; values in the second row are bacterial counts, the unit being cehs/µL.)

| | 16 | 8 | 4 | 2 | 1 | 0.5 | 0.25 | 0.12 | 0.06 | 0.03 | Positive control |
|---|---|---|---|---|---|---|---|---|---|---|---|
| Living cell counts | 880 | 1876 | 10876 | 10549 | 10654 | 10971 | 10278 | 10167 | 11426 | 11879 | 12576 |

Drug susceptibility testing results of Acinetobacter baumannii to Meropenem:
Results of the rapid drug susceptibility testing of living cells: MIC = 0.12; results of conventional drug susceptibility testing: MIC = 0.12.

See Table 12 for detailed data of the rapid drug susceptibility testing of living cells.

Table 12 (The values in the first row are the concentrations of the antibiotic drug, the unit being µg/mL; values in the second row are bacterial counts, the unit being cells/µL.)

| | 16 | 8 | 4 | 2 | 1 | 0.5 | 0.25 | 0.12 | 0.06 | 0.03 | Positive control |
|---|---|---|---|---|---|---|---|---|---|---|---|
| Living cell counts | 979 | 1003 | 1561 | 1479 | 1490 | 1514 | 1570 | 1574 | 6495 | 7959 | 8632 |

It can be seen from the results in Tables 7 to 12 that difference in an acceptable range was exhibited of the MIC obtained by live bacteria counting method (60% of the positive count value as the threshold to determine the MIC) of the preferred drugs as required by CLSI for the five clinically common bacterial strains selected for antibiotics susceptibility testing, as compared with the conventional drug susceptibility testing method by visual observation after 24 h, thus proving the feasibility of the resistance counting method for bacterial drug susceptibility testing and consistency with conventional methods.

The above description is only a preferred embodiment of the present disclosure and should not be considered as limiting the disclosure. The present disclosure may be subject to various changes and variations for those skilled in the art. Any modification, equivalent replacement, improvement, etc. made within the spirit and principles of the present disclosure shall all be included in the scope of protection of the present disclosure.

## Claims

1. A method for measuring inhibition of bacteria by an antibacterial drug, comprising:
adding a predetermined concentration of an antibacterial drug to bacteria to be detected and setting as a mixture of bacteria and drug; while also setting the bacteria to be detected without the antibacterial drug as a positive control;
when a first determined duration is reached from the time when the antibacterial drug is added, obtaining the current number of the bacteria in the mixture of bacteria and drug and the current number of the bacteria in the positive control;
determining that the antibacterial drug at the predetermined concentration inhibits or partially inhibits or does not inhibit the bacteria based on the ratio of the current number of the bacteria in the mixture of bacteria and drug to the current number of the bacteria in the positive control.

2. The method according to claim 1, when the ratio of the current number of the bacteria in the mixture of bacteria and drug to the current number of the bacteria in the positive control equals to a first predetermined threshold, it is determined that the antibacterial drug of the predetermined concentration inhibits the bacteria.

3. The method according to claim 2, wherein the first predetermined threshold is any one value between 0 and 0.6.

4. The method according to claim 3, wherein the first predetermined threshold is any one value between 0 and 0.4.

5. The method according to claim 2, when the ratio of the current number of the bacteria in the mixture of bacteria and drug to the current number of the bacteria in the positive control equals to a second predetermined threshold, it is determined that the antibacterial drug of the predetermined concentration partially inhibits the bacteria, but inhibition is not achieved;
when a second determined duration is reached from the time when the antibacterial drug is added, obtaining a second current number of the bacteria in the mixture of bacteria and drug and a second current number of the bacteria in the positive control, wherein the second predetermined duration is longer than the first predetermined duration;
when the ratio of the second current number of the bacteria in the mixture of bacteria and drug to the second current number of the bacteria in the positive control equals to the first predetermined threshold, it is determined that the antibacterial drug at the predetermined concentration inhibits the bacteria.

6. The method according to claim 5, when the ratio of the current number of the bacteria in the mixture of bacteria and drug to the current number of the bacteria in the positive control is greater than a second predetermined threshold, it is determined that the antibacterial drug at the predetermined concentration does not inhibit the bacteria.

7. The method according to claim 1, wherein the first predetermined duration is any one value between 0 and 1.5 hours, and the first predetermined duration does not equal to 0 hour.

8. The method according to claim 5, wherein the second predetermined threshold is any one value between 0.6 and 0.8.

9. The method according to any one of claims 1-8, wherein a resistance counting method is used for obtaining the current number of the bacteria in the mixture of bacteria and drug and the current number of the bacteria in the positive control.

10. The method according to claim 9, wherein the method for measuring inhibition of bacteria by the antibacterial drug comprises the following steps:
a. preparing the bacteria: inoculating the bacteria on a culture medium and incubating at a temperature between 20 degrees Celsius (°C) and 40 degrees Celsius (°C) for 15-24 hours and for later use;
b. preparing the mixture of bacteria and drug and the positive control, and incubating at a temperature between 20°C and 40°C.
c. after the first predetermined duration or the second predetermined duration, using the resistance counting method to obtain the current number or the second current number of the bacteria in the mixture of bacteria and drug or the current number or the second current number of the bacteria in the positive control;
d. when the ratio of the current number or the second current number of the bacteria in the mixture of bacteria and drug to the current number or second current number of the bacteria in the positive control equals to any one value between 0 and 0.4, it is determined that the antibacterial drug at the predetermined concentration inhibits the bacteria.

11. The method according to claim 10, wherein:
in the step a, the bacteria is inoculated on a blood agar culture medium and incubated at 37 degrees Celsius (°C) for 18 hours; and/or
in the step b, the mixture of bacteria and drug and the positive control are prepared and incubated at 37°C; and/or
in the step c, the first predetermined duration is 0.5 hour or 1 hour or 1.5 hours; the second predetermined duration is 2 hours or 2.5 hours or 3 hours.

12. The method according to any one of claims 1-8, wherein the current number of the bacteria in the mixture of bacteria and drug and the current number of the bacteria in the positive control is obtained by using a flow cytometry method or a microscopic method for counting bacteria or a counter measuring method or an electric counter counting method or a live cell counting method or a weighing method of cell weight.

13. The method according to claim 12, wherein the current number of the bacteria in the mixture of bacteria and drug and the current number of the bacteria in the positive control is obtained by a bacterial counting device, the bacterial counting device comprising:
a sampling module for obtaining a sample of bacteria to be counted;
a counting cell module comprising: an aperture, a front cell, a rear cell and electrodes, wherein the front cell and the rear cell are connected through the aperture, one of the electrodes being on each side of the aperture, the pressure on the liquid between the front cell and the rear cell being vacuum, the vacuum being used to make the sample of bacteria to be counted to pass from the front cell through the aperture into the rear cell;
a circuit control system for determining the number of bacteria in the sample of bacteria to be counted based on a pulse signal when the pulse signal generated on both sides of the aperture is detected, wherein the pulse signal is used to indicate that bacteria in the sample of bacteria to be counted have passed through the aperture.

14. The method according to claim 13, wherein the circuit control system comprises:
a first processor for detecting the pulse signal, transmitting the pulse signal to a processing equipment, and obtaining the number of bacteria in the sample of bacteria to be counted sent by the processing equipment, wherein the number of bacteria in the sample of bacteria to be counted is obtained on the basis of bacterial feature data indicated by the pulse signal; or
a second processor for detecting the pulse signal and determining the number of bacteria in the sample of bacteria to be counted on the basis of the bacterial feature data indicated by the pulse signal.

15. The method according to claim 13, wherein the circuit control system comprises:
a first power circuit for supplying a constant current to the aperture through the electrodes, wherein the pulse signal is a pulse signal triggered by one or more of the bacteria passing through the aperture while the constant current is supplied to the aperture; or
a second power circuit for supplying a constant voltage to the aperture through the electrodes, wherein the pulse signal is a pulse signal triggered by one or more of the bacteria passing through the aperture while the constant voltage is supplied to the aperture.

16. The method according to any one of claims 13-15, wherein the diameter of the aperture is a diameter within a first target diameter range, wherein the first target diameter range is used to allow only one bacterium at a time to pass through the aperture when the bacterium in the sample of bacteria to be counted passes through the aperture; or
the diameter of the aperture is a diameter within a second target diameter range, wherein the second target diameter range is used to allow a plurality of bacteria to pass through the aperture at a time as bacteria in the sample of bacteria to be counted pass through the aperture.

17. The method according to claim 16, wherein, in the case where the diameter of the aperture is a diameter within the first target diameter range, the diameter of the aperture is 30 microns to 70 microns, and/or, the aperture has a length of 30 microns to 100 microns.

18. The method according to claim 17, wherein, in the case where the diameter of the aperture is a diameter within the first target diameter range, the diameter of the aperture is 40 microns to 60 microns, and/or, the aperture has a length of 40 microns to 70 microns.

19. The method according to claim 18, wherein, in the case where the diameter of the aperture is a diameter within the first target diameter range, the diameter of the aperture is 50 microns, and/or, the aperture has a length of 50 microns.

20. The method according to claim 13, wherein the current number of the bacteria in the mixture of bacteria and drug and the current number of the bacteria in the positive control are obtained by a following bacterial counting method, comprising:
adding the sample of bacteria to be counted into the counting cell module, wherein the counting cell module comprises: an aperture, a front cell, a rear cell and electrodes, the front cell and the rear cell being connected through the aperture, the pressure on the liquid between the front cell and the rear cell being vacuum, the vacuum being used to make the sample of bacteria to be counted to pass from the front cell through the aperture into the rear cell, one of the electrodes being on each side of the aperture, and with the electrodes energized, there being a predetermined resistance between the two sides of the aperture;
detecting the presence of a pulse signal generated on both sides of the aperture due to a change in the resistance between the two sides of the aperture, wherein the pulse signal is used to indicate that bacteria in the sample of bacteria to be counted have passed through the aperture;
in the case that a pulse signal generated on both sides of the aperture is detected, obtaining the number of bacteria in the sample of bacteria to be counted as determined on the basis of the pulse signal.

21. The method according to claim 20, wherein, the obtaining the number of bacteria in the sample of bacteria to be counted as determined on the basis of the pulse signal comprises:
transmitting the pulse signal to a processing equipment, and obtaining the number of bacteria in the sample of bacteria to be counted sent by the processing equipment, wherein the number of bacteria in the sample of bacteria to be counted is obtained as determined on the basis of the bacterial feature data indicated by the pulse signal; or
determining the number of bacteria in the sample of bacteria to be counted on the basis of bacterial feature data indicated by the pulse signal.

22. The method according to claim 19 or 20, wherein the diameter of the aperture is a diameter within a first target diameter range, wherein the first target diameter range is used to allow only one bacterium at a time to pass through the aperture when bacteria in the sample of bacteria to be counted pass through the aperture; or
the diameter of the aperture is a diameter within a second target diameter range, wherein the second target diameter range is used to allow a plurality of bacteria to pass through the aperture at a time as bacteria in the sample of bacteria to be counted pass through the aperture.

23. A bacterial counting device, comprising:
a sampling module for obtaining a sample of bacteria to be counted;
a counting cell module comprising: an aperture, a front cell, a rear cell and electrodes, wherein the front cell and the rear cell are connected through the aperture, one of the electrodes being on each side of the aperture, the pressure on the liquid between the front cell and the rear cell being vacuum, the vacuum being used to make the sample of bacteria to be counted to pass from the front cell through the aperture into the rear cell;
a circuit control system for determining the number of bacteria in the sample of bacteria to be counted based on a pulse signal when the pulse signal generated on both sides of the aperture is detected, wherein the pulse signal is used to indicate that bacteria in the sample of bacteria to be counted have passed through the aperture.

24. The device according to claim 23, wherein the circuit control system comprises:
a first processor for detecting the pulse signal, transmitting the pulse signal to a processing equipment, and obtaining the number of bacteria in the sample of bacteria to be counted sent by the processing equipment, wherein the number of bacteria in the sample of bacteria to be counted is obtained on the basis of bacterial feature data indicated by the pulse signal; or
a second processor for detecting the pulse signal and determining the number of bacteria in the sample of bacteria to be counted on the basis of the bacterial feature data indicated by the pulse signal.

25. The device according to claim 23, wherein the circuit control system comprises:
a first power circuit for supplying a constant current to the aperture through the electrodes, wherein the pulse signal is a pulse signal triggered by one or more of the bacteria passing through the aperture while the constant current is supplied to the aperture; or
a second power circuit for supplying a constant voltage to the aperture through the electrodes, wherein the pulse signal is a pulse signal triggered by one or more of the bacteria passing through the aperture while the constant voltage is supplied to the aperture.

26. The device according to any one of claims 23-25, wherein the diameter of the aperture is a diameter within a first target diameter range, wherein the first target diameter range is used to allow only one bacterium at a time to pass through the aperture when bacteria in the sample of bacteria to be counted passes through the aperture; or
the diameter of the aperture is a diameter within a second target diameter range, wherein the second target diameter range is used to allow a plurality of bacteria to pass through the aperture at a time as bacteria in the sample of bacteria to be counted pass through the aperture.

27. The device according to claim 26, wherein, in the case where the diameter of the aperture is a diameter within the first target diameter range, the diameter of the aperture is 30 microns to 70 microns, and/or, the aperture has a length of 30 microns to 100 microns.

28. The device according to claim 27, wherein, in the case where the diameter of the aperture is a diameter within the first target diameter range, the diameter of the aperture is 40 microns to 60 microns, and/or, the aperture has a length of 40 microns to 70 microns.

29. The device according to claim 28, wherein, in the case where the diameter of the aperture is a diameter within the first target diameter range, the diameter of the aperture is 50 microns, and/or, the aperture has a length of 50 microns.

30. A bacterial counting method, **characterized by** comprising:
adding a sample of bacteria to be counted into a counting cell module, wherein the counting cell module comprises: an aperture, a front cell, a rear cell and electrodes, the front cell and the rear cell being connected through the aperture, the pressure on the liquid between the front cell and the rear cell being vacuum, the vacuum being used to make the sample of bacteria to be counted to pass from the front cell through the aperture into the rear cell, one of the electrodes being on each side of the aperture, and with the electrodes energized, there being a predetermined resistance between the two sides of the aperture;
detecting the presence of a pulse signal generated on both sides of the aperture due to a change in the resistance between the two sides of the aperture, wherein the pulse signal is used to indicate that bacteria in the sample of bacteria to be counted have passed through the aperture;
in the case that a pulse signal generated on both sides of the aperture is detected, obtaining the number of bacteria in the sample of bacteria to be counted as determined on the basis of the pulse signal.

31. The method according to claim 30, wherein, the obtaining the number of bacteria in the sample of bacteria to be counted as determined on the basis of the pulse signal comprises:
transmitting the pulse signal to a processing equipment, and obtaining the number of bacteria in the sample of bacteria to be counted sent by the processing equipment, wherein the number of bacteria in the sample of bacteria to be counted is obtained as determined on the basis of the bacterial feature data indicated by the pulse signal; or
determining the number of bacteria in the sample of bacteria to be counted on the basis of bacterial feature data indicated by the pulse signal.

32. The method according to claim 30 or 31, wherein the diameter of the aperture is a diameter within a first target diameter wherein the first target diameter range is used to allow only one bacterium at a time to pass through the aperture when bacteria in the sample of bacteria to be counted passes through the aperture; or
the diameter of the aperture is a diameter within a second target diameter range, wherein the second target diameter range is used to allow a plurality of bacteria to pass through the aperture at a time as bacteria in the sample of bacteria to be counted pass through the aperture.
